# EUROPEAN PATENT SPECIFICATION

(11) **EP 3 426 273 B1**
(45) Date of publication and mention of the grant of the patent: **21.04.2021**
(21) Application number: 17710217.5
(22) Date of filing: 10.03.2017
(51) Int. Cl.: A23L 33/105, A61K 9/00, A61K 9/20, A61K 31/015, A61K 31/085, A61K 31/09, A61K 31/34, A61K 31/341, A61K 31/355, A61K 31/365, A61K 31/385, A61K 36/15, A61K 45/06, A61P 11/00, A61P 15/08, A61P 29/00

(54) **SUBLINGUAL COMPOSITIONS COMPRISING NATURAL EXTRACTS AND USES THEREOF**
SUBLINGUALE ZUSAMMENSETZUNGEN MIT NATÜRLICHEN EXTRAKTEN UND VERWENDUNGEN DAFÜR
COMPOSITIONS SUBLINGUALES COMPRENANT DES EXTRAITS NATURELS ET LEURS UTILISATIONS

(30) Priority: 11.03.2016 EP 16305269
(43) Date of publication of application: 16.01.2019
(73) Proprietor: Harmonic Pharma, 54500 Vandoeuvre les Nancy (FR)
(72) Inventor: KARABOGA, Arnaud, Sinan, 67300 Schiltigheim (FR); GEGOUT, Stéphane, 54000 Nancy (FR); SOUCHET, Michel, 91190 Gif Sur Yvette (FR)
(74) Representative: Cabinet Becker et Associés
(86) International application number: PCT/EP2017/055620
(87) International publication number: WO 2017/153555

(56) References cited:
- EP-A1- 2 143 435
- WO-A1-2014/083171
- US-A1- 2002 061 854
- COSENTINO M ET AL: "Immunomodulatory activity of the lignan 7-hydroxymatairesinol potassium acetate (HMR/lignan@?) extracted from the heartwood of Norway spruce (Picea abies)", INTERNATIONAL IMMUNOPHARMACOLOGY, ELSEVIER, AMSTERDAM, NL, vol. 10, no. 3, 1 March 2010 (2010-03-01), pages 339-343, XP026919584, ISSN: 1567-5769, DOI: 10.1016/J.INTIMP.2009.12.005 [retrieved on 2009-12-11]
- SALEEM ALI BANIHANI: "Testosterone in Males as Enhanced by Onion (Allium Cepa L.)", BIOMOLECULES, vol. 9, no. 2, 21 February 2019 (2019-02-21), page 75, XP55733398, DOI: 10.3390/biom9020075
- MEGHAN A. BROWN ET AL: "Montmorency tart cherry ( Prunus cerasus L.) supplementation accelerates recovery from exercise-induced muscle damage in females", EUROPEAN JOURNAL OF SPORT SCIENCE, vol. 19, no. 1, 28 July 2018 (2018-07-28), pages 95-102, XP55733404, UK ISSN: 1746-1391, DOI: 10.1080/17461391.2018.1502360
- Aldo E. Calogero ET AL: "Conservative Nonhormonal Options for the Treatment of Male Infertility: Antibiotics, Anti-Inflammatory Drugs, and Antioxidants", BioMed Research International, vol. 2017, 1 January 2017 (2017-01-01), pages 1-17, XP55733405, ISSN: 2314-6133, DOI: 10.1155/2017/4650182
- WILLIAN DA SILVA ET AL: "Effect of green tea extract supplementation on exercise-induced delayed onset muscle soreness and muscular damage", PHYSIOLOGY AND BEHAVIOR, vol. 194, 1 October 2018 (2018-10-01), pages 77-82, XP55733407, GB ISSN: 0031-9384, DOI: 10.1016/j.physbeh.2018.05.006
- Maria Gammone ET AL: "Marine Bioactives and Potential Application in Sports", Marine Drugs, vol. 12, no. 5, 30 April 2014 (2014-04-30) , pages 2357-2382, XP55733408, DOI: 10.3390/md12052357
- Linda Grievink ET AL: "Dietary intake of antioxidant (pro)-vitamins, respiratory symptoms and pulmonary function: the MORGEN study", Thorax, vol. 53, 1 January 1998 (1998-01-01), pages 166-171, XP55733409,
- Ashok Kumar Grover ET AL: "Benefits of antioxidant supplements for knee osteoarthritis: rationale and reality", Nutrition Journal, vol. 15, no. 1, 1 December 2015 (2015-12-01), XP55733411, DOI: 10.1186/s12937-015-0115-z
- AHMED ISMAEEL ET AL: "Resistance Training, Antioxidant Status, and Antioxidant Supplementation", INTERNATIONAL JOURNAL OF SPORT NUTRITION & EXERCISE METABOLISM, vol. 29, no. 5, 1 September 2019 (2019-09-01), pages 539-547, XP055733413, US ISSN: 1526-484X, DOI: 10.1123/ijsnem.2018-0339
- STEPHEN J. IVES ET AL: "Effects of a combined protein and antioxidant supplement on recovery of muscle function and soreness following eccentric exercise", JOURNAL OF THE INTERNATIONAL SOCIETY OF SPORTS NUTRITION, vol. 14, no. 1, 3 July 2017 (2017-07-03), XP55733416, DOI: 10.1186/s12970-017-0179-6
- Gitte S. Jensen ET AL: "Consumption of Dried Apple Peel Powder Increases Joint Function and Range of Motion", JOURNAL OF MEDICINAL FOOD, vol. 17, no. 11, 1 November 2014 (2014-11-01), pages 1204-1213, XP55733417, US ISSN: 1096-620X, DOI: 10.1089/jmf.2014.0037
- LYALL K A ET AL: "Short-term blackcurrant extract consumption modulates exercise-induced oxidative stress and lipopolysaccharide-stimulated inflammatory responses", AMERICAN JOURNAL OF PHYSIOLOGY-REGULATORY , INTEGRATIVE AND COMPARATIVE PHYSIOLOGY, AMERICAN PHYSIOLOGICAL SOCIETY, US, vol. 297, no. 1, 1 July 2009 (2009-07-01), pages R70-R81, XP008148453, ISSN: 0363-6119, DOI: 10.1152/AJPREGU.90740.2008 [retrieved on 2009-04-29]
- MARTIN-HIDALGO ET AL: "Antioxidants and Male Fertility: from Molecular Studies to Clinical Evidence", ANTIOXIDANTS, vol. 8, no. 4, 5 April 2019 (2019-04-05), page 89, XP55733419, DOI: 10.3390/antiox8040089
- SENER TARIK EMRE ET AL: "Resveratrol treatment may preserve the erectile function after radiotherapy by restoring antioxidant defence mechanisms, SIRT1 and NOS protein expressions", INTERNATIONAL JOURNAL OF IMPOTENCE RESEARCH, STOCKTON, BASINGSTOKE, GB, vol. 30, no. 4, 5 July 2018 (2018-07-05), pages 179-188, XP036582924, ISSN: 0955-9930, DOI: 10.1038/S41443-018-0042-6 [retrieved on 2018-07-05]
- Shan Wang ET AL: "The Role of Antioxidant Enzymes in the Ovaries", Oxidative Medicine and Cellular Longevity, vol. 2017, 1 January 2017 (2017-01-01), pages 1-14, XP55733424, US ISSN: 1942-0900, DOI: 10.1155/2017/4371714
- Isabelle Romieu ET AL: "Antioxidant Supplementation and Respiratory Functions among Workers Exposed to High Levels of Ozone", American Journal of Respiratory and Critical Care Medicine, vol. 158, no. 1, 1 July 1998 (1998-07-01), pages 226-232, XP55733427, ISSN: 1073-449X, DOI: 10.1164/ajrccm.158.1.9712053
- T. WHYAND ET AL: "Pollution and respiratory disease: can diet or supplements help? A review", RESPIRATORY RESEARCH, vol. 19, no. 1, 2 May 2018 (2018-05-02), XP55733430, DOI: 10.1186/s12931-018-0785-0

## Description

### FIELD OF THE INVENTION

The present invention relates to the field of natural extracts and compositions comprising said extracts. The invention particularly relates the use of such compositions as a food supplement.

### BACKGROUND OF THE INVENTION

Since the industrial revolution, the air that we breathe is greatly degraded, notably polluted by strong oxidants substances, and this change in the atmosphere, commonly called "air pollution" has terrible consequences for humans and environment such as respiratory issues that affect millions of people worldwide. Further environment factors contribute to the badness respiratory such as exposure to an allergen (for example, dust mites, cockroaches, animal dander, and mold), exposure to cigarette smoke, low air quality due to traffic pollution or high ozone level, exposure to indoor volatile compounds (for example formaldehyde), exposure to phthalates present in PVC and exposure to high levels of endotoxin, environment risks from professional sources such as agricultural works, civil engineering, mining sector. WO 2014/083171 A1 discloses a knot-wood extract comprising at least one lignan selected from the list comprising (-)-hydroxymatairesinol (two epimers; 7S,8R,8'R and 7R,8R,8'R), (-)(7S, 8R, 8'R)-hydroxymatairesinol, (+)-lariciresinol, (-)-secoisolariciresinol, liovil and any epimers thereof. It is useful for treating a β2 adrenergic receptor related disease, preferably pulmonary diseases such as COPD; pre-term labour; neurological disorders; cardiac disorders; fibrosis; inflammation; atopic dermatitis; rheumatoid arthritis; thrombosis; allergies; ophthalmic diseases; urological disorders; gastrointestinal disorders; metabolic disorders and cancers. US 2002/061854 A1 discloses a pharmaceutical preparation comprising an effective amount of hydroxymatairesinol, a geometric isomer or a stereoisomer thereof, or a pharmaceutically acceptable salt thereof, in combination with a pharmaceutically acceptable carrier, wherein said agent is either the sole lignan present or the most abundant single component of a lignan mixture, and wherein said carrier is a solid carrier or a liquid carrier. It is useful in methods for prevention of cancers, certain non-cancer, hormone-dependent diseases and/ or cardiovascular diseases in a person, based on administering of hydroxymatairesinol. Preferably, it is extracted from Picea abies. COSENTINO M ET AL: "Immunomodulatory activity of the lignan 7-hydroxymatairesinol potassium acetate extracted from the heartwood of Norway spruce (Picea abies)", INTERNATIONAL IMMUNOPHARMACOLOGY, vol. 10, no. 3, 1 March 2010 (2010-03-01), pages 339-343, ISSN: 1567-5769, is a study of the immunomodulatory activity of the lignan 7-hydroxymatairesinol potassium acetate extracted from the heartwood of Norway spruce (Picea abies). The pharmacological profile of the lignan 7-hydroxymatairesinol includes chemopreventive effects, antioxidant properties, and mild pro-estrogenic activity. EP 2 143 435 A1 discloses a method for the isolation of secoisolariciresinol and dihydroquercetin from wood, preferably from Abies trees, wherein the crushed wood of the knot zone is extracted with a non-splitting mixture of an organic solvent with water at a solvent content of 50-75% to obtain the extract containing secoisolariciresinol and dihydroquercetin, and the extract is treated to remove the solvent to obtain the final mixture containing secoisolariciresinol and dihydroquercetin. The resulting products are useful for the production of biologically active additives, chemical and pharmaceutical products.

Given this increasingly significant air pollution, there is a need to develop novel substances and novel formulations in order to improve the respiratory wellness.

### SUMMARY OF THE INVENTION

In this context, the inventors have identified novel compositions comprising a part of branch extracts, preferably from resinous trees, which are enriched in polyphenols and exhibit antioxidant and/or anti-inflammatory properties. More particularly, the inventors have surprisingly demonstrated that such extracts formulated in sublingual compositions had an improved antioxidant efficacy while maintaining an anti-inflammatory effect, and were suitable for improving the respiratory wellness, relieving joint pain and/or muscular pain and cramps and/or improving endurance, and improving libido and/or fertility. The claimed invention is strictly defined in the appended set of claims.

The present invention therefore relates to a sublingual composition comprising at least about 10 % by weight of a part of branch extract from a mixture of Abies alba and Picea abies, said part of branch extract comprises at least about 50 %, preferably at least 70 %, more preferably at least 90 % by weight of polyphenols and less than about 10 % by weight of terpenes, relative to the total weight of the extract.

The polyphenols of the part of branch extract comprises:
- from about 25 % to about 50 % by weight of hydroxymatairesinol,
- from about 5 % to about 15 % by weight of lariciresinol, and
- from about 5 % to about 15 % by weight of secoisolariciresinol,
relative to the total weight of the extract.

In a further particular embodiment, the polyphenols of said extracts are selected from the group consisting of hydroxymatairesinol, lariciresinol, secoisolariciresinol and any epimers thereof.

In a preferred embodiment, the sublingual composition of the invention, comprises from about 10% to about 50 % by weight of the part of branch extract as disclosed herein, relative to the total weight of the composition.

In a particular embodiment, the sublingual composition further comprises thickeners, sweeteners, disintegrating agents, dispersing agents, lubricants, and/or flow agents.

In a more preferred embodiment, the sublingual composition of the invention comprises:
- from about 10 % to about 50 %, preferably from about 10 % to about 40 %, more preferably from about 10 % to about 20 %, by weight of said extract;
- from about 40 % to about 90 %, preferably from about 50 % to about 80 %, by weight of microcrystalline cellulose;
- from about 0.01 % to about 2 %, preferably from about 0.50 % to about 1.50 %, by weight of reticulated sodium carboxymethyl-cellulose; and
- from about 0.01 % to about 2 %, preferably from about 0.50 % to about 1.50 %, by weight of magnesium stearate;
relative to the total weight of the composition.

In a further particular embodiment, the sublingual composition as defined herein further comprises at least one active ingredient for respiratory wellness and/or at least one dietary supplement.

Preferably, the at least one active ingredient for respiratory wellness is chosen among an extract from *Abies, Abies siribica, Acerola, Achellea millefolium, Allium ursinum, Althaea officinalis, Andrographis paniculata,* anise, apple, *Artemisia cina, Arum korolkowii, Asperula humifusa, Astragalus, Avenca, Aalsam peru, Aiebersteinia multifidi,* bishop's weed, black spruce, black seed, blueberry, borage, *Brazilian peppertree,* butterbur, *Camellia sinensis,* carapia, catnip, caraway, *Cinnamomum verum, Cinnamomum zeylanicum,* citrus, *Citrus paradisi, Citrus sinenseschamomile,* chaparral, chlorella, *Coleus forskohlii,* coltsfoot, copaiba, *Curcuma longa, Cymbopognon citratus, Cychopelarrium intybus,* dandelion, danshen, *Drosera, echinacea,* embauba, *English ivy,* eucalyptus, *Cucapyptus radiate, Espinheira, Evening primrose, Filipendula ulmaria,* fir, forsythia, *Fucus vesiculosus,* garlic, gelsemium, *Genipa americana, Gotu kola,* ginkgo, ginseng, great plantain, grindelia, *Guaco, mikania glomerata spreng,* guandu, hawthorn, *Hedera helix,* horseradish, horsetail, *Indian frankincense,* juazeiro, *Juniperus seravschanica, Justicia paniculata,* kalanchoe, khella, kiwi, larch, lavender, lavandula, lobelia, *Malpighia glabra, Melaleuca quinquenervia, Malva neglecta, Origanum majorana, Marrubium vulgare,* moringa, myrtus, nettle, *Nigella sativa, Origanum vulgare, Panax ginseng,* pau d'arco, pelargonium, melaleuca, mentha, *Mentha piperita, Origanum majorana, Pelargonium sidoides,* peppermint, *Perilla, picrorhiza,* pine, piper, *Phyllanthus emblica, Plantago major,* plantain, *Plectranthus barbatus, Primula veris,* quillaja, red clover, rosemary, rosmarinus, *Salvia officinalis,* samambaia, schisandra, *Siraitia grosvenorii, Soia isoflavoni, Sophora japonica,* sorrel, soy, *Spirulina platensis,* sweet orange, *Syzygium aromaticum, Taraxacum officinale, Terminalia chebula, Terminalia belerica, Thymus serpyllum, Thymus vulgaris,* tomato, *Tussilágo fárfara,* vassourinha, *Verbascum thapsus, Viburnum lantana,* white horehound, and *Yerba santa,* and/or chosen among alpha-linolenic acid, alpha-lipoic acid, arabinogalactan, arginine, balsam tolu, beta-glucan, bifidobacteria, brewer's yeast, bromelain, caffeine, choline, coenzyme Q-10, cordyceps, eicosapentaenoic acid (EPA), forskolin, fish oil, gamma-aminobutyric acid (GABA), glutathione, green mold, green-lipped mussel, inositol, lactobacillus, linoleic acid, lutein, lycopene, matico, N-acetylcysteine (NAC), pyridoxine, propolis, pycnogenol, quail egg, quercetin, resveratrol, saffron, selenium, serrapeptase, shark liver, umckaloabo, whey protein, yogurt, and zeaxanthin.

Preferably, the at least one dietary supplement is ascorbic acid, a vitamin or a mineral chosen among copper, calcium, magnesium, zinc, vitamin A, vitamin B1, vitamin B2, vitamin B3, vitamin B5, vitamin B6, vitamin B8, vitamin B9, vitamin B12, vitamin C, vitamin D, vitamin E, vitamin K, iron, iodine, manganese, and molybdenum.

Another object of the invention is a non-therapeutic use of a sublingual composition as defined herein as a food supplement, preferably for improving the respiratory wellness, and/or for improving libido and/or fertility. A further object of the invention is a non-therapeutic use of a sublingual composition as defined herein as a food supplement, preferably as sport food supplement, especially for relieving joint pain and/or muscular pain and cramps and/or improving endurance.

The present invention also concerns a kit comprising a sublingual composition (e.g. food supplement composition) as defined herein and a package leaflet or user instructions including the information that said composition is to be used for the respiratory wellness, and/or for joint pain and/or muscular pain and cramps and/or endurance, and/or libido, and/or fertility.

### DETAILED DESCRIPTION OF THE INVENTION

The inventors have identified novel sublingual compositions comprising a part of branch extract, preferably from resinous trees, which is enriched in polyphenol, and have demonstrated by several methods that such extracts exhibited an improved antioxidant and antiradical activity compared to antioxidant reference compounds such as pycnogenol, α-tocopherol (vit. E), and trolox (vit. E like), but also to its main metabolites such as enterolactone and enterodiol. The part of branch extracts of the invention further exhibit anti-inflammatory properties illustrated by the inhibitory effect of said extract against arachidonate 5-lipoxygenase (5-lipoxygenase or 5-LOX or 5-LO) and prostaglandin-endoperoxide synthase 2 (cyclooxygenase-2 or COX-2), and by the inhibitory effect of hydroxymatairesinol (HMR) against interleukin-1β (IL-1β), tumor necrosis factor-α (TNF- α), 5-lipoxygenase, and prostaglandin-endoperoxide synthase 2. The present invention relates to a sublingual composition as disclosed herein comprising a part of branch extract, preferably from at least one resinous tree, comprising at least about 50 %, preferably at least 70 %, more preferably at least 90 % by weight of polyphenols and less than about 10 % by weight of terpenes, relative to the total weight of the extract.

### Part of branch extract

Typically, the extract of the invention is obtained by a standard extraction process from part of branches of resinous trees comprising the following steps of (a) extracting, (b) separating liquid / solid (filtering), (c) concentrating, and (d) drying. Preferably, the part of branch extract is a dry extract.

The part of branch extract is a part of branch from *Abies alba* and/or *Picea abies.*

As used herein the "part of branch" includes the part of the tree excluding the trunk. A part of branch includes for instance leaves, rods, bark, stems, buds, and knot-wood. In a particular embodiment, the « part of branch » excludes knot-wood. In a further particular embodiment, the "part of branch" includes leaves, rods, bark, stems, and buds and is especially devoid of knot-wood.

The part of branch extract of the sublingual compositions of the invention comprises at least about 50 %, 60 %, 70 %, preferably 80 %, more preferably at least about 90 % by weight of polyphenols and less than about 10 %, 8 %, 6 %, preferably less than about 5 % by weight of terpenes, relative to the total weight of the extract.

Polyphenols are a structural class of chemicals characterized by the presence of large multiples of phenol structural units. Polyphenols can also be called "lignan" when they are found in plants. As an example of polyphenol or lignan found in resinous trees, the following may be cited:
- Hydroxymatairesinol (HMR) having the following formula: 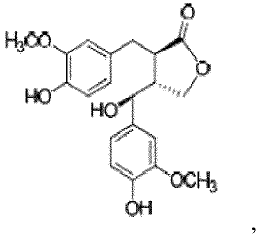
- Lariciresinol having the following formula: 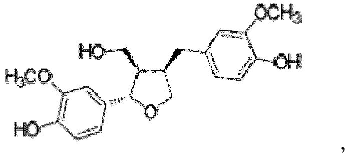
- Secoisolariciresinol having the following formula: 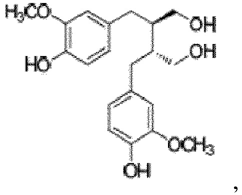 and
- Liovil having the following formula:

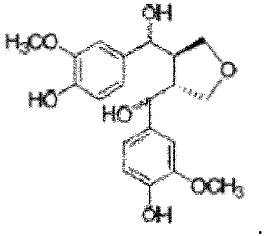

Terpenes are a large and diverse class of organic compounds, produced by a variety of plants, particularly resinous trees. Terpenes are derived biosynthetically from units of isoprene having the molecular formula C₅H₈ and represented by the following formula:

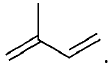

As main terpenes found in resinous trees, o-Guaicol, terpin-hydrate, vanillin, 2,5-dimethylbenzenebutanoic acid, conifeyl alcohol, tumerone, juvabione, dehydrojuvabione, manool, and eucalyptol (1,8-cineol) may be cited.

The part of branch extract of the sublingual compositions of the invention having an improved antioxidant effect is enriched with polyphenols compared to terpenes, and particularly, comprises less than about 10 % by weight of terpenes, relative to the total weight of the extract.

As used herein, the term "about" will be understood by a person of ordinary skill in the art and will vary to some extent on the context in which it is used. If there are uses of the term which are not clear to persons of ordinary skill in the art given the context in which it is used, "about" will mean up to plus or minus 10 %, preferably 5 % of the particular term.

Preferably, the polyphenol of the part of branch extract of the invention are selected from the group consisting of hydroxymatairesinol (HMR), lariciresinol, secoisolariciresinol and any epimers thereof.

As used herein, the term "epimers" refers to diastereoisomers that differ in configuration of only one stereogenic center. The term "diastereoisomers" is a class of stereoisomers non-superimposable, non-mirror images of one another. For instance, "epimers of hydroxymatairesinol" refer to the diastereoisomers 7S, 8R, 8'R and 7R, 8R, 8'R as illustrated in the following formula:

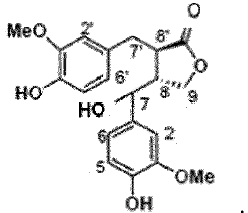

The part of branch extract of the invention comprises:
- from about 25 % to about 50 % by weight of hydroxymatairesinol,
- from about 5 % to about 15 % by weight of lariciresinol, and
- from about 5 % to about 15 % by weight of secoisolariciresinol,
relative to the total weight of the extract.

The part of branch extract of the sublingual compositions of the invention can further comprise any other compound found in resinous trees in small amount such as terpenes as mentioned above and, typically, eucalyptol (1,8-cineol). However, as eucalyptol is known for its toxicity, it is preferably present in small amounts, preferably less than about 10 % by weight, more preferably less than about 2 %, and even more preferably less than about 1 %, by weigh relative to the total weight of the extract.

In a preferred embodiment, the part of branch extract comprises less than 1 % by weight of eucalyptol, relative to the total weight of the extract.

As demonstrated by examples, the part of branch extract of the sublingual compositions of the invention enriched in polyphenols, i.e. comprising at least about 90 % by weight of polyphenols, has an improved antioxidant activity, in addition to its anti-inflammatory activity, and is therefore suitable for a respiratory wellness application. Such part of branch extract is also suitable for sports practice such as jogging and running, thanks to its improved antioxidant activity allowing to relieve or prevent joint paint and/or improving endurance.

### Sublingual composition

The present invention concerns a sublingual composition comprising a part of branch extract as defined herein. Particularly, the composition of the invention can further comprise any excipients, including diluent and/or carriers. Such excipients do not produce an adverse, allergic or other untoward reaction when administered to an animal, preferably a human. For human administration, compositions should meet sterility, pyrogenicity, general safety and purity standards as required for instance by FDA (Food and Drug Administration) Office of Biologics standards.

In the context of the present invention, the composition is suitable for sublingual administration. Examples of forms adapted to such administration include, but are not limited to tablets, capsules, pills, powders, granules, ointments, pastes, creams, spray, gels, liposomal forms, patches such as transdermal patches or mucoadhesive patches. The composition of the invention is administrated by a sublingual route allowing thereby to avoid the metabolism of the polyphenols of the extract such as by digestive tract in order to maintain an efficient and optimized antioxidant effect. As demonstrated by examples of the present application, a part of branch extract of the invention has a greater antioxidant and antiradical effect compared to its main metabolites i.e. enterodiol and enterolactone. According to this sublingual administration, the polyphenols of the part of branch extract are not metabolized by digestive tract and exhibit an improved antioxidant or antiradical activity while having an anti-inflammatory activity.

Examples of excipients, diluent and/or carriers suitable for a sublingual administration include, without limitation, water, phosphate buffer saline (PBS), anaeorobic phosphate buffer saline, sodium bicarbonate, coloring agents, thickener agents, sweeteners, coating agents, diluting agents, binding agents, disintegrated agents, dispersing agents, lubricant agents, flow agents, etc.

Coloring agents include for instance titanium dioxide (E171), iron dioxide (E172), and brilliant black BN (E151). Thickeners include for instance, without limitation, glycerol monostearate and starch, such as corn starch, maize starch, potato starch, and rice starch. Sweeteners include for instance, without limitation, aspartame, agave syrup, stevia extract, mannitol, sorbitol, xylitol, lactitol, erythritol, isomalt, maltitol, saccharin, sucralose, sucrose, fructose, neotame, acesulfame potassium, advantame, cyclamates. Coating agents include for instance, without limitation, refined colza oil, soya oil, peanut oil, soya lecithin and fish gelatin. Diluting agents include for instance, without limitation, lactose, monohydrated lactose and starch. Binding agents include for instance, without limitation, povidone, pregelatinized starch, gums, saccharose, polyethylene glycol (PEG) 4000 and PEG 6000. Disintegrated agents include for instance, without limitation, microcrystalline cellulose, sodium carboxymethyl starch such as sodium carboxymethyl starch type A, and reticulated sodium carboxymethyl-cellulose. Dispersing agents include for instance, without limitation, polyoxyethyleneglycol distearate. Lubricant agents include for instance, without limitation, magnesium stearate and talc. Flow agents include for instance, without limitation, colloidal anhydrous silica and colloidal silica dioxide.

Particularly, the sublingual compositions of the invention further comprise thickeners, sweeteners, disintegrating agents, dispersing agents, lubricants, and/or flow agents.

In a preferred embodiment, the thickener agent is corn starch.

In a further preferred embodiment, the sweetener agent is fructose.

In a further preferred embodiment, the disintegrating agents are microcrystalline cellulose and/or reticulated sodium carboxymethyl-cellulose.

In a further preferred embodiment, the lubricants are magnesium stearate and/or talc, more preferably magnesium stearate.

In a further preferred embodiments, the dispersing agent is polyoxyethyleneglycol distearate.

In a particular embodiment the composition of the invention comprises at least about 10 %, preferably from about 10 to 50 %, more preferably from about 10 % to about 40 %, even more preferably from about 10 % to about 35% by weight of the part of branch extract as defined in the present application, relative to the total weight of the composition.

A preferred composition of the invention comprises:
- from about 10 % to about 50 %, preferably from about 10 % to about 40 %, more preferably from about 10 % to about 35 % by weight of the part of branch extract as above defined;
- from about 40 % to about 90 %, preferably from about 50 % to about 80 %, more preferably from about 60 % to about 70 % by weight of microcrystalline cellulose;
- from about 0.01 % to about 5 %, preferably from about 0.50 % to about 2 %, more preferably from about 0.50 % to about 1.50 %, even more preferably from about 0.80 % to about 1.30 % by weight of reticulated sodium carboxymethyl-cellulose; and
- from about 0.01 % to about 5 %, preferably from about 0.50 % to about 2 %, more preferably from about 0.60 % to about 1.80 % by weight of magnesium stearate; relative to the total weight of the composition.

A more preferred composition of the invention comprises:
- 50-150 mg of the part of branch extract as defined herein;
- 200-250 mg, preferably about 230 mg of microcrystalline cellulose;
- 1-10 mg, preferably about 4 mg of reticulated sodium carboxymethyl-cellulose;
- 30-70 mg, preferably about 50 mg of polyoxyethyleneglycol distearate;
- 1-10 mg, preferably about 3 mg of magnesium stearate; and
- 1-10 mg, preferably about 3 mg of talc.

A further more preferred composition of the invention comprises:
- 30-100 mg, preferably 40-60 mg, more preferably about 50 mg of the part of branch extract as defined herein;
- 150-200 mg, preferably about 165 mg of microcrystalline cellulose;
- 1-10 mg, preferably about 3 mg of reticulated sodium carboxymethyl-cellulose;
- 30-70 mg, preferably about 50 mg of polyoxyethyleneglycol distearate;
- 1-10 mg, preferably about 4 mg of magnesium stearate;
- 1-5 mg, preferably about 3 mg of colloidal silica dioxide;
- 10-50 mg, preferably about 20 mg of fructose; and
- 5-30 mg, preferably about 10-20 mg, more preferably about 17 mg of corn starch.

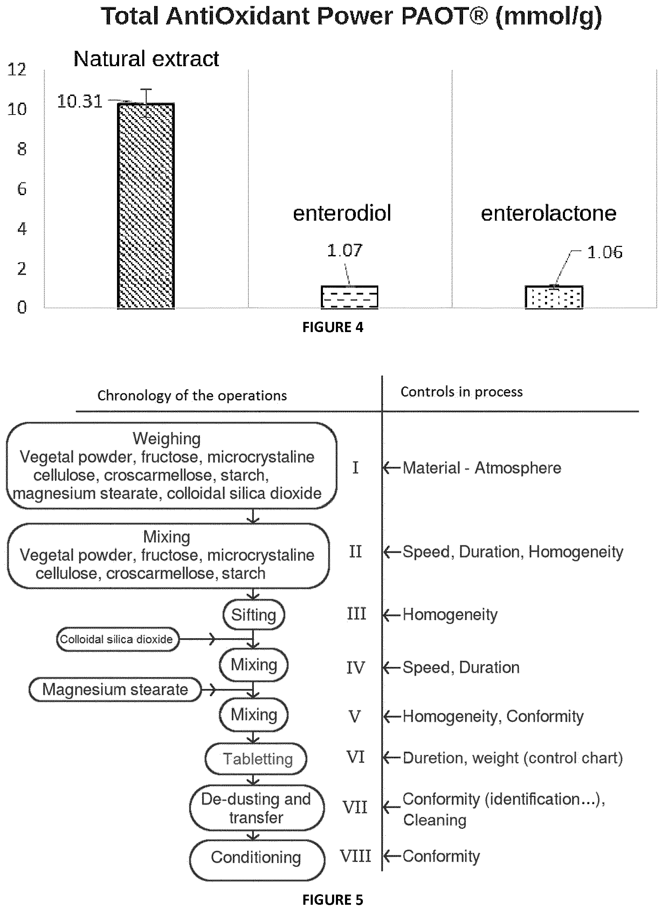

The sublingual compositions of the invention are prepared by mixing the ingredients as above defined followed by a step of tableting by direct compression as illustrated in Figure 5.

### Application

Thanks to the improved antioxidant and anti-inflammatory activities of the part of branch extract as disclosed herein, sublingual compositions comprising said extract may be used as a food supplement.

In a further particular embodiment, the sublingual compositions of the invention further comprise at least one dietary supplement or food supplement.

As used herein, a "food supplement" or a "dietary supplement" is intended to provide nutrients that may otherwise not be consumed in sufficient quantities. According to the FDA, food supplements are products which are not pharmaceutical drugs and must also meet criteria as defined in the Dietary Supplement Health and Education Act of 1994. They are generally administrated by oral and/or sublingual administration. Food supplement include for instance, without limitation, vitamins, minerals, fatty acids, amino acids, herbal medicine, etc.

In a further particular embodiment, the sublingual compositions of the invention further comprise at least one dietary supplement such as minerals, vitamins and ascorbic acid. In a preferred embodiment, the at least one dietary supplement is chosen among copper, calcium, magnesium, zinc, vitamin A, vitamin B1, vitamin B2, vitamin B3, vitamin B5, vitamin B6, vitamin B8, vitamin B9, vitamin B12, vitamin C, vitamin D, vitamin E, vitamin K, iron, iodine, manganese, molybdenum, and ascorbic acid.

A first application is a non-therapeutic use of the sublingual compositions of the invention, preferably as a food supplement, for improving the respiratory wellness.

As used herein the expression "respiratory wellness" also means promoting an optimum pulmonary function and preventing alteration of the function of the pulmonary system.

The invention therefore concerns the use of a sublingual composition of the invention for improving the respiratory wellness, preferably the non-therapeutic use thereof. Indeed, in this context, the subject to be treated is healthy, i.e. does not have any respiratory or pulmonary disease such as asthma, COPD (chronic obstructive pulmonary disease), chronic bronchitis, emphysema, pneumonia, lung cancer.

The invention further concerns a method for improving the respiratory wellness in a subject, preferably a human, comprising administering by sublingual route to said subject an effective amount of a sublingual composition as defined herein.

As used herein, the expression "effective amount" means the minimal amount of the composition of the invention to obtain sufficient anti-inflammatory and antioxidant activities allowing to improve the respiratory wellness in a subject. More specifically, the effective amount of the part of branch extract or the composition of the invention is ranging from about 1 ng/kg to about 10 g/kg of body weight. Preferably, the effective amount of the part of branch extract or the composition of the invention is ranging from about 10 ng/kg to about 1 g/kg, more preferably from about 100 ng/kg to about 100 mg/kg, and even more preferably from about 500 ng/kg to about 50 mg/kg of body weight.

The invention may further relate to the sublingual composition of the invention for use for improving respiratory wellness. In this context, the subject to be treated has or is susceptible to develop a respiratory or pulmonary disease.

In a particular embodiment, the sublingual compositions of the invention further comprise at least one active ingredient for respiratory wellness. More particularly, the active ingredient for respiratory wellness is chosen among an extract of plants such as *Abies, Abies siribica, Acerola, Achellea millefolium, Allium ursinum, Althaea officinalis, Andrographis paniculata,* anise, apple, *Artemisia cina, Arum korolkowii, Asperula humifusa, Astragalus, Avenca, Aalsam peru, Aiebersteinia multifidi,* bishop's weed, black spruce, black seed, blueberry, borage, *Brazilian peppertree,* butterbur, *Camellia sinensis,* carapia, catnip, caraway, *Cinnamomum verum, Cinnamomum zeylanicum,* citrus, *Citrus paradisi, Citrus sinenseschamomile,* chaparral, chlorella, *Coleus forskohlii,* coltsfoot, copaiba, *Curcuma longa, Cymbopognon citratus, Cychopelarrium intybus,* dandelion, danshen, *Drosera, echinacea,* embauba, *English ivy,* eucalyptus, *Cucapyptus radiate, Espinheira, Evening primrose, Filipendula ulmaria,* fir, forsythia, *Fucus vesiculosus,* garlic, gelsemium, *Genipa americana, Gotu kola,* ginkgo, ginseng, great plantain, grindelia, *Guaco, mikania glomerata spreng,* guandu, hawthorn, *Hedera helix,* horseradish, horsetail, *Indian frankincense,* juazeiro, *Juniperus seravschanica, Justicia paniculata,* kalanchoe, khella, kiwi, larch, lavender, lavandula, lobelia, *Malpighia glabra, Melaleuca quinquenervia, Malva neglecta, Origanum majorana, Marrubium vulgare,* moringa, myrtus, nettle, *Nigella sativa, Origanum vulgare, Panax ginseng,* pau d'arco, pelargonium, melaleuca, mentha, *Mentha piperita, Origanum majorana, Pelargonium sidoides,* peppermint, *Perilla, picrorhiza,* pine, piper, *Phyllanthus emblica, Plantago major,* plantain, *Plectranthus barbatus, Primula veris,* quillaja, red clover, rosemary, rosmarinus, *Salvia officinalis,* samambaia, schisandra, *Siraitia grosvenorii, Soia isoflavoni, Sophora japonica,* sorrel, soy, *Spirulina platensis,* sweet orange, *Syzygium aromaticum, Taraxacum officinale, Terminalia chebula, Terminalia belerica, Thymus serpyllum, Thymus vulgaris,* tomato, *Tussilágo fárfara,* vassourinha, *Verbascum thapsus, Viburnum lantana,* white horehound, and *Yerba santa.*

The active ingredient for respiratory wellness may also be chosen among alpha-linolenic acid, alpha-lipoic acid, arabinogalactan, arginine, balsam tolu, beta-glucan, bifidobacteria, brewer's yeast, bromelain, caffeine, choline, coenzyme Q-10, cordyceps, eicosapentaenoic acid (EPA), forskolin, fish oil, gamma-aminobutyric acid (GABA), glutathione, green mold, green-lipped mussel, inositol, lactobacillus, linoleic acid, lutein, lycopene, matico, N-acetylcysteine (NAC), pyridoxine, propolis, pycnogenol, quail egg, quercetin, resveratrol, saffron, selenium, serrapeptase, shark liver, umckaloabo, whey protein, yogurt, and zeaxanthin.

In a preferred embodiment, the sublingual compositions of the invention comprise at least one active ingredient for respiratory wellness and at least one dietary supplement.

A second application is a non-therapeutic use of the sublingual compositions of the invention, preferably as a food supplement, more preferably as a sport food supplement. More particularly, the sport food supplement is for relieving and/or preventing pain, in particular joint and/or muscular pain, for preventing cramps, and/or for improving endurance and/or for preventing doping behavior.

Physical exercise is accompanied by a significant increase in the oxidation of nutrients to meet energy needs. Many physiological systems and multiple biochemical interactions come into play during the exercise. Yet the most important interactions, and those that limit performance, are between the cardiorespiratory system and skeletal muscles. Indeed, a multiplication of oxygen consumption by ten or twenty fold in comparison with rest occurs.

Endurance can be defined as the capacity to maintain a long-term and intense effort. Is obvious that endurance is essential to the sportsman. Without it, his abilities are limited. The improvement of endurance is one of the main objectives of sportsmen, even amateurs. Sport is indeed a whole package associating technique, strength and endurance. Improving endurance allows not only to maintain an effort longer but also to increase comfort and pleasure in sports activities. A good endurance allows the cyclist to ascend a pass with pleasure and the long-distance runner to roam several hours. In competitions, endurance makes the difference between two sportsmen of similar skill: the athlete who endures the most fatigue will handle better at the end of the contest, and is more likely to be victorious. Practicing endurance is also of a certain interest for health. Physical exercise, when regularly practiced, protects from high blood pressure, excess in sugars and fats, three factors responsible for the clogging of arteries (and therefore cardiovascular diseases). Moreover, it helps fighting other diseases, as osteoporosis, type 2 diabetes and some cancers. It is also known that sport strengthens the immune system, increase heart volume (thus its efficiency) and empowers respiratory faculties. Finally, it maintains muscular mass and avoids muscles atrophy.

In training, as in competition, endurance sportsmen are confronted with energy losses and free radical generation which slow down their performances, deteriorate their recovery and can cause cramps or fatigue. This free radical generation generally comes along with an increase of antioxidant defenses, but the oxidizing stress can exceed adaptation capacities. Therefore, it is coherent to help the body with antioxidants to improve performance. Also, Rena and Torresb (Brain Res. Reb., 2009, 60(1), 57-64) have shown the role of IL-1β during pain and inflammation and have suggested that blockade of IL-1β could be a strategic approach for relieving joint pain. Furthermore, Samad et al. (Nature, 2001, 410, 471-475) have demonstrated that the inhibition of IL-1β reduces centrally generated inflammatory pain hypersensitivity and Martin et al. (American Journal of Physiology, 2006, 291, 1344-1348) have suggested that increased brain IL-1β plays an important role in fatigue after muscle-damaging exercise.

In a particular embodiment, the sublingual compositions of the invention further comprise at least one active ingredient, e.g., useful as food sport supplement. More particularly, the active ingredient can be selected from the group consisting of vitamin B1, vitamin B2, vitamin B3, vitamin B5, vitamin B6, vitamin B8, vitamin B9, vitamin B12, vitamin C, vitamin D, vitamin D3, vitamin E, vitamin H, vitamin K, vitamin PP, bromelain, carnitine, casein protein, choline bitartrate, chondroitin, copper, creatine, chromnium, dimethylglycine (DMG), glucosamine, idebedone, iron, iode, isoleucine, L-glycine, leucine, magnesium, maltodextrin, manganese, ornithine, Q10, selenium, spiruline, taurine, valine, whey protein, zinc, *Allium sativum,* arnica, artichoke, arthrospira, astragalus (Astragalus membranaceus), bananas, beer yeast, beta-carotene, betula, blueberry, caffeine, *Centella asiatica, Cinchona officinalis,* chlorella, cordyceps, *Cordyceps sinensis,* cynorhodon, *Echinacea purpurea, Echinacea angutifolia, Echinacea pallid,* eleutherococcus, fumaria, fraxinus, harpagophytum, harpagophytum procumbens, *Hippophae rhamnoides, Hycdrocotyle asiatica,* ginger, *Ginko biloba,* ginseng, goji berries, green tea, guarana, (*Paullinia cupana*), jujube, *Lepidium meyenii,* lycopene, *Malpighia glabra,* nettle, olive leaf, orthosiphon, panax ginseng, *Phoenix dactylifera,* pollen, *Rhodiola rosea, Rosmarinus officinalis, Salix alba, Sambucus nigra,* saturjea, *Schisandra chinensis, Sphaeria sinenses,* spirulina, thistle, thymus, trigonelle fenugrec, *Tribulus terrestris,* urtica, valerian (*Valeriana officinalis, Valeriane radix*), and *Withania somnifera.* In a preferred embodiment, the sublingual compositions of the invention further comprise selenium.

The sublingual compositions of the invention may also be taken by a subject before and/or after a sport practice, especially in a prospect for joint health, joint support, typically for relieving and/or preventing joint pain, and/or preventing muscular pain and cramps, and/or for improving endurance.

The sublingual compositions of the invention can be administered once (for an immediate effect) or during a period of time (for long-term effect), for instance during a week, a couple of weeks, one month or a couple of months. The curing period may be determined based on training cycles.

An object of the invention is therefore a non-therapeutic use of a sublingual composition of the invention, preferably as a sport food supplement, in particular for relieving joint pain, and/or preventing muscular pain and cramps, and/or improving endurance.

As mentioned herein, an "athlete" is a person who practice sports, such as running, jogging, and any sports requiring a physical activity. In this context there is no difference between a professional athlete and an athlete practicing physical activity as a hobby. The sublingual compositions of the invention are suitable for a professional athlete or a normal athlete practicing sports as a hobby.

A further object of the invention is a sublingual composition of the invention for use for relieving and/or preventing joint pain. In this context, the subject or the athlete to be treated has or is susceptible to develop joint pain, and up to more serious diseases such as joint fusion, joint infection, osteoarthritis, and rheumatoid arthritis, etc.

For a sport application, the sublingual compositions of the invention can further comprise at least one dietary supplement or food supplement as defined above and/or at least one active ingredient for respiratory wellness as above defined.

A third application is a non-therapeutic use of a sublingual composition of the invention, preferably as a food supplement, for improving libido.

Indeed, it is well-known oxidative stress has a negative impact on libido. As it is known that oxidative stress can be relieved with antioxidants, then they help dilate blood vessels, improve blood circulation and combat erectile dysfunction. Indeed, stimulation of the sex cells of the genital area by relaxing the blood vessels in that part of the individual is called the corpora cavernosa. The corpora cavernosa is a spongy ball tiny nerves surrounded by blood vessels located in the penis and clitoris. For men, an erection and best sensations are authorized by the proper functioning of the corpora cavernosa. In women, sexual pleasure and achieving an orgasm are often dependent on the activity of the cavernous body of the clitoris. The corpus cavernosum becomes active when engorged with blood during periods of sexual excitement; its stimulation depends on the relaxation of genital muscles. Nitric oxide and its metabolite, cyclic GMP, are the two dominant biochemicals which induce relaxation of genital muscles and, consequently, increase the blood flow to the corpus cavernosum.

In particular, recent studies have shown that antioxidants can reverse endothelial dysfunction thus improving blood flow to the penis. They do this by neutralizing free radicals that cause oxidative stress and endothelial damage in the relevant blood vessels. Researchers have shown that antioxidant supplements may improve erectile function. Also, Eggert-Kruse (Fertil Steril., 2007, 87, 810-823) have discussed the role of IL-1β in patients suffering depression, which is well known for reducing libido.

In a particular embodiment, the sublingual compositions of the invention further comprise at least one active ingredient, e.g., useful as improving libido. More particularly, the active ingredient can be selected from the group consisting of vitamin A, vitamin B, vitamin B3, vitamin B6, vitamin B9, vitamin C, vitamin D, vitamin E, arginine, CDP choline, CFM Nitro, dimethylglycine (DMG), dioscine, diosgenin, eleutherococcus, glutamine, gracilline, isoleucine, L-Arginine, leucine, linoleic acid, L-lysine, L-norvaline, magnesium, nicotinamide adenine dinucleotide (NADH), pantothenic acid, protogracillin, selenium, silibinine, valine, zinc, *Aesculus hippocastanum,* achyranthe, *Agnuscastus, Angelicasinensis,* arthrospira,ashwagandha (*Withania somnifera*), astragalus, *Astragalus membranaceus, Avena sativa, Berberis vulgaris,* bioperine, caffeine, catuaba (*Erythroxylum catuaba*), capsicin, cayenne pepper, *Centella asiatica,* chilli pepper, cinnamon, *Cinnamomum camphora, Cinnanomum cassia,* cistanche, citrus, citrulline, cnidium, *Cnicus benedictus, Cnidium monnieri,* cordyceps, *Cornus mas, Cornus officinalis,* curcuma, cuscuta, *Cuscuta chinensis,* cynomorium stem, damiana (*Turnera aphrodisiaca*), *Dipsacus fullonum, Elettaria cardamomum,* epimedium (*Epimediumsagittatum*), epimede (horny goat weed, grandiflorum), eucommia, *Eucommia ulmoides, Eugenia caryophyllata, Eurycoma longifolia* (long jack root), *Erythroxylum vaccinifolium,* damiana (*Turnera diffusa*), fenugrec (*Trigonella foenum graecum*), *Flos carthami, Galium odoratum,* garlic, ginger, *Ginkgo biloba,* ginseng, goji berries, green tea, hawthorn (*Fructus crataegi*), *Herba cistanches,* heracleum, hippocampus, *Hippophae rhamnoides,* histidine, icariin, *Ignatia Amara,* jujube, kiwi, *Lactuca virosa, Lepidium Meyenii, Ligusticum wallichii, Mandragora officinarum,* maca (*Lepidium meyenii*), *Momordica charantia, Mucuna pruriens, Muira puama* (*Acanthea virilis, Ptychopetalum olacoides*), *Myrciaria dubia,* niacin, *Origanum dictamnus, Paeonia lactiflora, Panax ginseng, Paullinia cupana,* pepper, piperine, phenylethylamine, *Polygala tenuifolia,* protodioscine, *Radix Ligustici Wallichii,* red berries, *Rhizoma cucurmae, Rhodolia rosea, Richeria grandis,* rosa, *Rosa damascena,* rosemary, sabal, saffron, satureja, saw palmetto berry (*Fructus serenoae*), schizandra, *Schisandra chinensis,* scrophularia, *Sichuan aconite, Silybum marianum, Smilax aspera,* spirulina, taurine, terrestris, tongkat ali (*Eurycoma longifolia*), *Tribulus terrestris, Trigonelle fenugrec,* urtica, vanilla, vitex agnus-castus, wheat germ, *Wolfiporia extensa,* and yohimbe.

A further object of the invention is a sublingual composition of the invention for use for improving libido.

A fourth application is a non-therapeutic use of a sublingual composition of the invention, preferably as a food supplement for improving fertility, especially in men. Sperm are protected by a membrane formed from unsaturated fatty acids, which may be destroyed by the free radicals generated by oxidative stress. It is this oxidative stress causes infertility in part by nearly one-third of infertile men. Moreover, a surplus of free radicals can prevent the synthesis of nitric oxide and thereby cause of erectile dysfunction. Moreover, experiments conducted in 2005 showed that men consuming more antioxidants produce the largest volume of semen and the strongest sperm.

Also, Dousset et al. (Human Reproduction, 1997, 12, 1476-1479) have studied blood hormonal status in male infertility and have observed an increase of IL-1β in patients with infertility.

In a particular embodiment, the sublingual compositions of the invention further comprise at least one active ingredient, e.g., useful as improving fertility. More particularly, the active ingredient can be selected from the group consisting of vitamin B1, vitamin B2, vitamin B3, vitamin B6, vitamin B8, vitamine B12, vitamin D, vitamin D3, vitamin E, vitamin C, vitamin K, astaxanthin, beta-carotene, bromelain, calcium, choline, chromium, copper, CoQ10, DHEA, folate, folic acid, iodine, inositol, L-acetyl cysteine, L-arginine, L-carnitine, L-glutathione, lipoic acid, manganese, magnesium, melatonin, N-acetyl cystein, octacosanol, omega 3 fatty acids, pantothenic acid, para-aminobenzoic acid, progesterone, red clover blossom, selenium, zinc, *Achillea millefolium, Angelica sinensis, Actaea racemosa, Arctium lappa, Agnus Castus* (*Vitex Agnus Castus*), *Aloe vera, Angelica sinensis, Artemisia vulgaris,* ashwagandha, *Asparagus racemosus, Avena sativa, Black Cohosh, Cimicifuga racemosa* (*Actaea racemosa*), citrus, dandelion, dong quai, *Eurycoma longifolia,* false unicorn, ginseng, panax, *Glycyrrhiza glabra,* honey, horse chesnut, *Ilybum marianum, Leonurus cardiaca, Lepidium meyenii* (MACA), *Linum usitatissimum,* lycopene, milk thistle, *Oenothera biennis, Petroselinum crispum, Polygonum multiflorum,* propolis, *Ptychopetalum olacoides,* raspherry leaf, red clover, red raspberry, royal jelly, saffron, *Sesamum indicum, Schisandra chinensis,* siberian ginseng, stinging Nettle, *Taraxacum officinale, Trifolium pratense, Tribulus terrestris, Urtica dioica, Vitex agnus-castus,* and *Withania somnifera.*

A further object of the invention is a sublingual composition of the invention for use for improving fertility.

Another object of the invention is a kit comprising a composition as defined herein and a package leaflet or user instructions including the information that said composition is to be used for the respiratory wellness, and/or for joint pain and/or muscular pain and cramps and/or endurance, and/or libido and/or fertility.

### FIGURES

**Figure 1****:** Evaluation of the anti-radical activity of the extract of the invention (natural extract), hydroxymatairesinol (HMR), enterodiol, enterolactone, and pycnogenol by DPPH method.
**Figure 2****:** Evaluation of the antioxidant activity of the extract of the invention (natural extract) and its main metabolites (enterodiol and enterolactone), expressed as equivalent quantity of trolox (square-patterned) and vitamin E (dotted line).
**Figure 3****:** Evaluation of the total antioxidant power of the extract of the invention (natural extract), pycnogenol, and hydroxymatairesinol (HMR), expressed as millimole of global antioxidant per gram of product, by Technology PAOT/POT®.
**Figure 4****:** Evaluation of the total antioxidant efficacy of the extract of the invention (natural extract) and its main metabolites (enterodiol and enterolactone), expressed as millimole of global antioxidant per gram of product, by technology PAOT/POT®.
**Figure 5****:** Diagram of manufacturing process for the tablets containing vegetable powder.
**Figure 6A****:** Evaluation of the anti-inflammatory activity of the extract of the invention by measuring the inhibitory effect of HMR (representative polyphenol compound of the extract of the invention) at 10, 30, and 100 µM towards TNF-α and IL-1β cytokines.
**Figure 6B** **and** **6C****:** Evaluation of the anti-inflammatory activity of the extract of the invention by measuring the inhibitory effect of HMR (representative polyphenol compound of the extract of the invention) at 10, 30, and 100 µM towards TNF-α (Figure 6B) and IL-1β (Figure 6C) cytokines after 5 µg/mL LPS induction.
**Figure 7****:** Evaluation of the anti-inflammatory activity of the extract of the invention by measuring the inhibitory effect of the extract at 10 µM and HMR at 10 µM towards 5-LO and COX-2.

Further aspects and advantages of the invention will be disclosed in the following experimental section.

### EXAMPLES

### Example 1: Sublingual compositions

### 1. Extraction process of the part of branch extracts.

54 kg of natural crude material made of *Picea abies* and *Abies alba* part of branch was placed in an extractor (e.g. filtering bottom extractor Lavergne type equipped with a nylon membrane) and was extracted using 15 % ethanol in water (v/v) as a solvent with a ratio material/solvent of 1/10 at 40 °C for 24 hours. Separation liquid/solid was obtained by use of a spin dryer. Solid extract was placed in said extractor and was extracted using 15 % ethanol in water (v/v) as a solvent with a ratio material/solvent of 1/10 at 40°C for 1 hour. Separation liquid/solid was obtained by use of a spin dryer. The two liquid extracts were combined and concentration of liquid extract was performed under vacuum at 60 °C with an evaporator (e.g. falling flow Wiegand type). Drying of the extracts was performed by atomization using a stainless steel spray dryer (e.g. atomization tower APV PSD 52 type) to give 3.88 kg of dry extract.

### 2. Process for the preparation of compositions of the invention

The equipment used for the manufacturing of the tablets may comprise a Turbula mixer, a tableting machine Frogerais equipped with convex punches 10 mms in diameter, a balance of precision Ohaus. The tableting is carried out by direct compression of the mixture of various excipients and plant extract.

As shown in Figure 5, the diagram of manufacturing of the composition B comprises a stage of weighing of the ingredients, a first stage of mixture followed by two successive mixtures, this one being followed with the additions of magnesium stearate and colloidal silica. Various controls carried out relate to the homogeneity of the mixture of powders, the weight of the tablets obtained, the tableting of the powder without the vegetable powder, and the angle of Haussner.

More specifically, the first mixing has a duration of 30 minutes. The 2^{nd} and 3^{rd} mixings have one duration respectively of 3 minutes. Industrially, it is possible that it is necessary to conceive a stage of sifting at the end of the various stages of mixtures.

The composition A is obtained according the process as illustrated in figure 5 using particular ingredients of the composition A.

### 3. Sublingual formulations

### 3.1 Sublingual composition A

| | |
|---|---|
| Part of branch extract from *Abies alba* and *Picea abies* | 50-150 mg |
| Microcrystalline cellulose | 230 mg |
| Reticulated sodium carboxymethyl-cellulose | 4 mg |
| Magnesium stearate | 3 mg |
| Talc | 3 mg |
| Polyoxyethyleneglycol distearate | 50 mg |

### 3.2 Sublingual composition B

| | |
|---|---|
| Part of branch extract from *Abies alba* and *Picea abies* | 50 mg |
| Microcrystalline cellulose | 166.70 mg |
| Reticulated sodium carboxymethyl-cellulose | 3.33 mg |
| Magnesium stearate | 4.36 mg |
| Fructose | 26.70 mg |
| Corn starch | 16.70 mg |
| Colloidal silica dioxide | 2.70 mg |

### Example 2: Evaluation of antioxidant and antiradical activities of the part of branch extracts of the invention.

### 2.1. DPPH (2,2-diphenyl-1-picrylhydrazyl) Assay

(Burda and Oleszek: "Antioxidant and anti-radical activities of flavonoids", J. Agric. Food Chem., 2001, 49, 2774-2779).

To study the anti-radical activity of the extract, DPPH test has been used. DPPH free radical has a violet color which turns to yellow when it is reduced in the presence of free-radical compounds. The color intensity, measured with the spectrophotometer, is inversely proportional to the anti-radical activity of the tested compounds. Different dilutions of honeybee samples dissolved in methanol were added to 3.9 ml of a 6.1×10⁻⁵ M DPPH• methanolic solution. The exact initial DPPH• concentration (C DPPH•) in the reaction medium was calculated by a calibration curve. The bleaching of DPPH• was monitored at 517 nm (Spectrophotometer) for 30 min against a blank constituted by the sample containing all reagents except DPPH•. The scavenging activity of the samples on the DPPH• was expressed as IC50 (mg/ml) and was extrapolated from a dose-response curve. The assays were performed in quadruplicate (n = 4) with the extract of the invention, hydroxymatairesinol (HMR), enterodiol, enterolactone, and pycnogenol® (French maritime pine bark extract, reference antioxidant compound). The anti-radical activity is expressed as a value of EC50 (the smaller EC50 value corresponds to the stronger antioxidant activity).

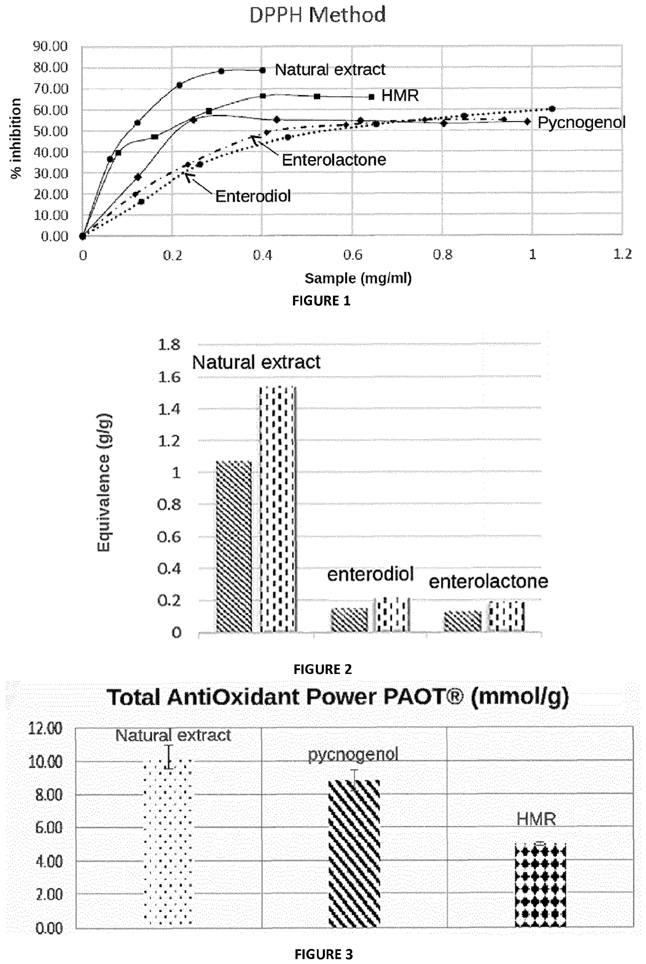

As displayed in Figure 1, the antioxidant activity of the part of branch extract of the invention is better than HMR, enterodiol, enterolactone, and pycnogenol. Indeed, the part of branch extract of the invention surprisingly shows an EC50 (Effective Concentration to reduce 50% of DPPH radicals) of 0.13 mg/ml which is smaller than EC50 of HMR (EC50 = 0.20 mg/ml) and pycnogenol (EC50 = 0.21 mg/ml).

In addition, the extract of the invention shows an EC50 of 0.13 mg/ml which is smaller than EC50 of its metabolites - i.e. enterodiol (EC50 = 0.28 mg/ml) and enterolactone (EC50 = 0.30 mg/ml).

### 2.2. Trolox Equivalent Antioxidant Capacity (TEAC) Assay

(Re R., Pellegrini N., Proteggente A., Pannala A., Yang M., Rice-Evans C.: "Antioxidant activity applying an improved ABTS radical cation decolorization assay", Free Radical Biology & Medicine, 1999, 26 (9), 1231-1237).

The total antioxidant activity of the extract of the invention derives from its ability to inhibit the radical ABTS • +, obtained from ABTS (ammonium salt of 2, 2 '-azinobis (3-ethylbenzothiazoline-acid 6-sulfonic) acid) compared to a reference antioxidant compound (pycnogenol®, α-tocopherol, Trolox) and its metabolites (enterodiol and enterolactone). ABTS radical cation (ABTS• +) was produced by reacting 7 mM ABTS stock solution with 2.45 mM potassium persulfate and allowing the mixture to stand in the dark at room temperature for 12-16h before being used. Next ABTS• + solution was diluted with PBS (Phosphate Buffer Saline) (pH 7.4) to an absorbance of 0.5 at 734 nm and equilibrated at 30°C. 200 µl of ABTS • + and (10, 20, 35, 50, 70, 80 µl) of product solutions (0.5 mg/L) was mixed and adjusted to 300 µl with buffer solution. After 15 min incubation, the optical density was measured. The assays were performed in quadruplicate (n = 4). Efficacy is expressed as a value of EC50 (the smaller value of EC50 corresponds to the stronger antioxidant activity). The natural extract of the invention surprisingly shows the smallest EC50 (Effective Concentration to remove 50% of ABTS at equilibrium) with a value of 0.020 mg/mL in that assay as displayed in Table 1.

**Table 1: EC50 values obtained in the TEAC assay**

| **Sample** | **Extract** | **enterodiol** | **enterolactone** | **pycnogenol** | **α-tocopherol** (vit. E) | **trolox** (vit. E like) |
|---|---|---|---|---|---|---|
| EC50 (mg/ml) | 0.02 | 0.14 | 0.17 | 0.030 | 0.031 | 0.022 |

In addition, Figure 2 emphasizes the striking discrepancy in antioxidant efficacy between the part of branch extract and its main metabolites. Indeed, antioxidant activity of 1 g of the natural extract is equivalent to 1.07 g of trolox (square-patterned) and 1.53 g of vitamin E (dotted line) whereas antioxidant activity of 1 g of enterolactone is equivalent to 0.13 g of trolox (square-patterned) and 0.19 g of vitamin E (dotted line), and antioxidant activity of 1 g of enterodiol is equivalent to 0.15 g of trolox (square-patterned) and 0.22 g of vitamin E (dotted line).

### 2.3. Oxygen Radical Absorbance Capacity (ORAC) Assay

(Ou B., Hampsch-Woodill M. and Prior R. L.: "Development and Validation of an Improved Oxygen Radical Absorbance Capacity Assay Using Fluorescein as the Fluorescent Probe", J. Agric. Food Chem., 2001, 49 (10), 4619-4626)

Measurement of antioxidant capacity by the ORAC method is based on the detection of fluorescence drop of fluorescein (FL) thanks to its reaction with peroxyl radical ROO *, in a food matrix containing antioxidant compounds. Fluorescence was read with an excitation wavelength of 485 nm and an emission filter of 528 nm. AAPH solution and FL were prepared in a phosphate buffer solution at 75 mM at pH 7.4. The control of this reaction is the trolox which is also prepared in the same buffer. Six different quantities (10, 20, 35, 50, 70, 80 µl) of product with concentration of 0.5 mg/L were placed in the wells of the microplate with FL and adjusted at the same volume with buffer solution (80 µl). The mixture was preincubated for 30 min at 37 °C, before rapidly adding the AAPH solution (220 µl) using a multichannel pipette. The microplate was immediately placed in the reader and the fluorescence recorded every 6 min for 240 min. A blank with FL and AAPH using sodium phosphate buffer instead of the antioxidant solution was used. The inhibition capacity was expressed as reference compound equivalents (µM), and is quantified by integrating of the area under the curve (AUC) of the fluorescein. The assays were carried out in quadruplicate (n = 4). Efficacy of the tested samples is expressed as reference ORAC units per gram of products (the higher value corresponds to the stronger antioxidant activity). Results are illustrated in Table 2.

**Table 2: Antioxidant activity of the part of branch extract of the invention, pycnogenol, HMR, enterodiol, and enterolactone. Values are expressed as equivalent units (µmol of reference compound per gram of tested product).**

| | **Extract** | **pycnogenol** | **HMR** | **enterodiol** | **enterolactone** |
|---|---|---|---|---|---|
| µmol Trolox/g tested product | 2400±500 | 1270±60 | 210±50 | 500±60 | 560±50 |
| µmol Vit. C/g tested product | 1300± 200 | 670±90 | 110±10 | 700±100 | 110±10 |
| µmol Vit. E/g tested product | 1200±170 | 630±70 | 105±10 | 600±80 | 105±10 |
| µmol β-carotene/g tested product | 310±20 | 165±5 | 27±2 | 165±5 | 30±2 |

The results show that the antioxidant activity of 1 gram of the part of branch extract is respectively equivalent to the total antioxidant power of 2400 µmol of trolox, 1300 µmol of vitamin C, 1200 µmol of vit. E (tocopherol), and 310 µmol of β-carotene. Surprisingly the antioxidant activity of the part of branch extract is largely greater than the antioxidant activity of 1 g of pycnogenol, 1 g of hydroxymatairesinol (HMR) and 1 g of the metabolites of the natural extract (enterodiol and enterolactone).

### 2.4. Total AntiOxidant Power (PAOT®) Assay (oxidative stress)

Technology PAOT/POT® (Total AntiOxidant Power/Total Oxidant Power) is based on the electrochemical nature of redox reactions: a balance between antioxidants and oxidants [www.ie-antioxydants.com]. The assays were carried out in quadruplicate (n = 4) (The higher value corresponds to the best antioxidant efficacy).

As displayed in Figure 3, the overall antioxidant efficacy of the natural extract (10.31 mmol/g equivalent of antioxidant) of the invention is surprisingly better than pycnogenol (8.82 mmol/g equivalent of antioxidant), and HMR (4.99 mmol/g equivalent of antioxidant).

Table 3 below summarizes values of antioxidant activity of the natural extract, pycnogenol, and HMR with regard to reference antioxidant compounds i.e. vitamin E (tocopherol), trolox, and β-carotene. Surprisingly, the part of branch extract shows the best antioxidant activity among the tested products. Actually, 1 gram of part of branch extract of the invention is equivalent to the antioxidant efficacy of 2.2 g of vitamin E, 1.29 g of trolox, and 5.53 g of β-carotene.

**Table 3: Antioxidant activities with regard to reference compounds.**

| | **Part of branch extract** | **pycnogenol** | **HMR** |
|---|---|---|---|
| **Vitamin E*** | 2.22 | 1.90 | 1.07 |
| **Trolox*** | 1.29 | 1.10 | 0.62 |
| **β-carotene*** | 5.53 | 4.74 | 2.68 |

| | | | |
|---|---|---|---|
| * gram of reference compound per gram of tested product | | | |

As illustrated in Figure 4, the discrepancy between efficacy of the part of branch extract of the invention and its main metabolites in that assay, expressed as millimole of global antioxidant per gram of product (measure of the global antioxidant feature of tested products; the higher value corresponds to the best antioxidant efficacy).

Indeed, the inventors have surprisingly shown that the total antioxidant efficacy of the part of branch extract (10.31mmol/g equivalent of antioxidant) was at least ten times greater than the antioxidant efficacy of its metabolites (enterodiol (1.07 mmol/g equivalent of antioxidant) and enterolactone (1.06 mmol/g equivalent of antioxidant)).

### 2.5. Conclusions

The inventors have surprisingly shown that the natural part of branch extract according to the invention had a greater antioxidant activity compared to reference compounds as pycnogenol, α-tocopherol (vit. E), and trolox (vit. E like), thereby demonstrating an improved efficacy for respiratory wellness, for relieving joint pain and/or muscular pain and cramps and/or for improving endurance, and for improving libido and/or fertility. The inventors have also surprisingly shown that the natural part of branch extract according to the invention had a greater antioxidant activity compared to its main metabolites (enterolactone and enterodiol), thereby demonstrating an improved efficacy when the composition is administered by a route avoiding metabolization such as sublingual route.

### Example 3: Evaluation of anti-inflammatory activity of HMR (representative polyphenol compound of the part of branch extracts of the invention).

Interleukin-1beta (IL-1beta) and tumor necrosis factor-alpha (TNF-alpha) mediate the development of numerous inflammatory diseases. IL-1beta and TNF-alpha therefore constitute suitable biomarkers to characterize the anti-inflammatory property of compounds of the part of branch extracts of the invention.

Peripheral blood mononuclear cells (PBMCs) were prepared from the peripheral blood of healthy donors (Etablissement Français du Sang) according to Kümmerle et al. (J Med Chem. 2012, 55, 7525-7545) with slight modifications. PBMCs were isolated using Histopaque gradient (Sigma), washed in Hanks' balanced salt solution (Sigma) and were then cultured in RPMI-1640 medium (Sigma) supplemented with 10% (v/v) fetal bovine serum (FBS) (PAA), 100 U/mL penicillin and 100 µg/mL streptomycin (PAA). PBMCs were seeded into 96-well plates (8×104 cells/well) and stimulated with 5 µg/mL LPS (Lipopolysaccharides, Sigma), into a total volume of 120 µL per well. HMR was re-suspended in DMSO and tested at 10, 30, and 100 µM (DMSO 1%) in duplicate. Cells without LPS stimulation were considered as bio-inactive control (basal level of cytokine). Cells incubation was realized at 37 °C (5% CO₂).

After 24 h, PBMCs supernatants were transferred into a 384-well plate. Cytokine detection was performed using HTRF technology (Cisbio bioassays) for TNF-α (62TNFPEC), IL-1β (62IL1PEC) according to supplier recommendations. Reading was performed after 3 h of incubation using Envision multi-labelled reader (PerkinElmer) using supplier recommended parameters.

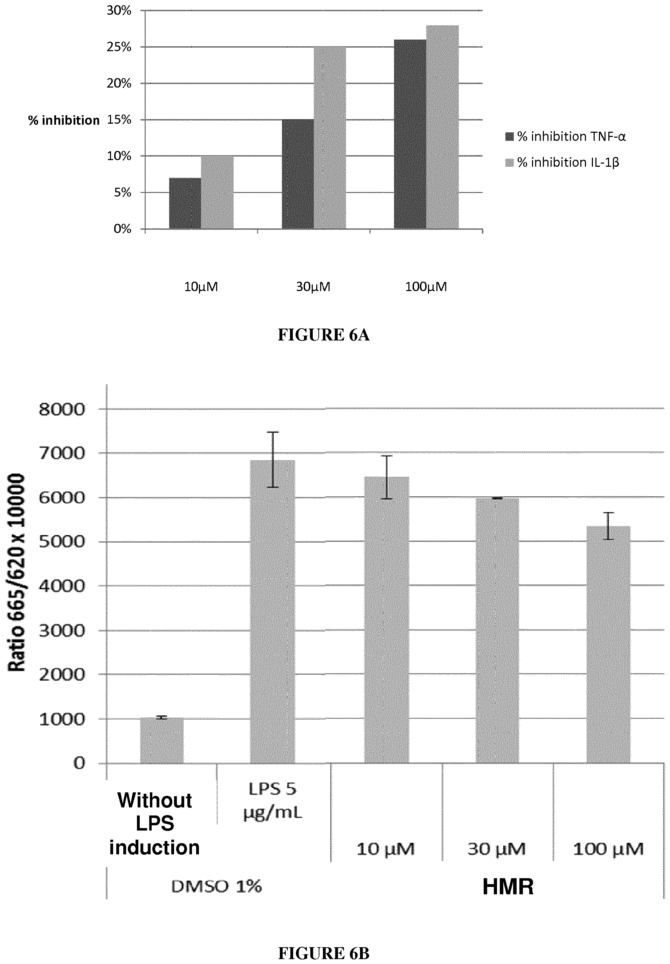

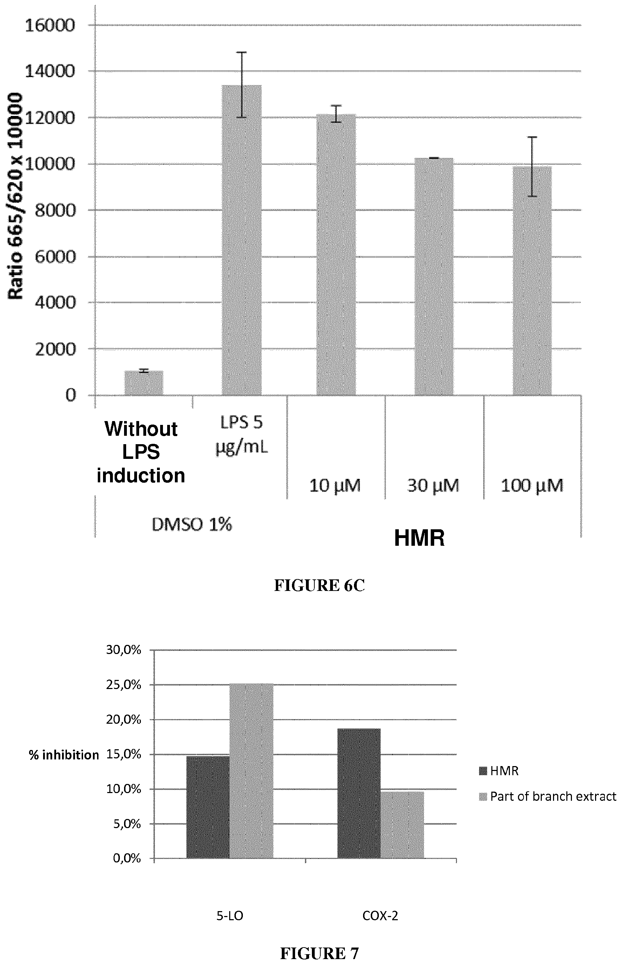

The results are represented in the table 4 below and in figures 6A, 6B, and 6C.

**Table 4: % inhibition TNF-α and IL-1β with HMR at 10, 30, and 100 µM**

| HMR concentration | % inhibition TNF-α (Standard deviation) | % inhibition IL-1β (Standard deviation) |
|---|---|---|
| 10 µM | 7 % (8 %) | 10 % (3 %) |
| 30 µM | 15 % (0 %) | 25 % (0 %) |
| 100 µM | 26 % (5 %) | 28 % (10 %) |

The results show the inhibitory effect of HMR against IL-1beta and TNF-alpha, thereby demonstrating an anti-inflammatory effect for the natural part of branch extract and sublingual compositions comprising such extract. Also, figures 6B and 6C particularly show a dose-dependent inhibitory effect for HMR against TNF-alpha and IL-1beta, respectively.

### Example 4: Evaluation of anti-inflammatory activity of HMR and a part of branch extract of the invention.

Prostaglandin-endoperoxide synthase 2 (cyclooxygenase-2; COX-2) and arachidonate 5-lipoxygenase (5-lipoxygenase; 5-LOX; 5-LO) are well known enzymes involved in inflammation.

### Human COX-2 inhibition assay:

The test compound, reference compound or water (control) were pre-incubated for 20 min at room temperature with the enzyme (≈ 0.2 µg) in a buffer containing 90 mM Tris-HCl (pH 8.0), 1.98 mM phenol and 1.02 µM hematine. Thereafter, the reaction was initiated by adding 2 µM arachidonic acid and the mixture was incubated for 5 min at room temperature. For basal control measurements, arachidonic acid was omitted from the reaction mixture. Following incubation, the reaction was stopped by the addition of 1 M HCl then 1 M Tris/HCl (pH 8.0) followed by cooling to 4°C. The fluorescence acceptor (d2 labeled PGE2) and the fluorescence donor (anti-PGE2 antibody labeled with europium Cryptate) were then added. After 120 min, the fluorescence transfer corresponding to the amount of residual PGE2 was measured at λex =337 nm, λem =620 nm and λem=665 nm using a microplate reader (Envision, Perkin Elmer). The enzyme activity was determined by dividing the signal measured at 665 nm by that measured at 620 nm (ratio).

The results are expressed as a percent inhibition of the control enzyme activity. The standard inhibitory reference compound is NS398, which was tested in each experiment at several concentrations to obtain an inhibition curve from which its IC50 value was calculated.

Reference: Glaser, K., Sung, M.L., O'neill, K., Belfast, M., Hartman, D., Carlson, R., Kreft, A., Kubrak, D., Hsiao, C.L. and Weichman, B. (1995), Etodolac selectively inhibits human prostaglandin G/H synthase 2 (PGHS-2) versus human PGHS-1. Eur. J. Pharmacol., 281: 107-111.

### Human 5-LO inhibition assay:

The test compound, reference compound or water (control) were preincubated for 5 min at room temperature with 10⁴ lysed cells/well in a buffer containing 50 mM Tris-HCl (pH 7.4), 5 mM CaCl₂, 2 mM EDTA, 1 µM ATP and 50 nM fluorescent probe dihydrorhodamine 123. The fluorescence intensity was then measured at λex=485 nm and λem=535 nm using a microplate reader (Envision, Perkin Elmer). This measurement at t=0 allowed the detection of any compound interference with the fluorimetric detection method at these wavelengths. Thereafter, the reaction was initiated by adding 25 µM of the substrate arachidonic acid and the mixture was incubated for 20 min at room temperature. The reaction was stopped by adding a buffer containing 67% acetonitrile and 0.2% acetic acid and the fluorescence intensity emitted by the reaction product rhodamine 123 was measured at the same wavelengths (t=20). The enzyme activity was determined by subtracting the signal measured at t=0 from that measured at t=20.

The results are expressed as a percent inhibition of the control activity. The standard inhibitory reference compound is NDGA, which was tested in each experiment at several concentrations to obtain an inhibition curve from which its IC₅₀ value was calculated.

Reference: ufahl, R.A., Kasten, T.P., Hills, R., Gierse, J.K., Reitz, B.A., Weinberg, R.A. and Masferrer, J.L. (2007). Development of a fluorescence-based enzyme assay of human 5-lipoxygenase. Anal. Biochem., 364: 204.

The results are presented in the table 5 below and in figure 7.

**Table 5: % inhibition of 5-LO and COX-2 with HMR (10µM) and a part of branch extract (equiv. 10µM)**

| Biomolecular target (reference compound) | % Inhibition | |
|---|---|---|
| | **HMR** | **Part of branch extract** |
| **5-LO** (NDGA; EC50=270nM) | 14.7 | 25.2 |
| **COX-2** (NS398; EC50=99nM) | 18.7 | 9.6 |

The results show the inhibitory effect of HMR and a part of branch extract against 5-LO and COX-2, thereby demonstrating an anti-inflammatory effect for the natural part of branch extract and sublingual compositions comprising such extract.

### Conclusions

The inventors have surprisingly shown that sublingual compositions according to the invention, in which polyphenols are not metabolized, had a greater antioxidant activity, while maintaining an anti-inflammatory activity. Such sublingual compositions exhibiting such double activity are thus suitable for improving respiratory wellness, for relieving joint pain and/or muscular pain and cramps, and/or for improving endurance, and for improving libido and/or fertility.

## Claims

1. A sublingual composition comprising at least about 10 % by weight of a part of branch extract from a mixture of *Abies alba* and *Picea abies,* relative to the total weight of the composition, wherein said part of branch extract comprises at least about 50 %, preferably at least 70 %, more preferably at least 90 % by weight of polyphenols and less than about 10 % by weight of terpenes, relative to the total weight of the extract, and wherein polyphenols comprise:
- from about 25 % to about 50 % by weight of hydroxymatairesinol,
- from about 5 % to about 15 % by weight of lariciresinol, and
- from about 5 % to about 15 % by weight of secoisolariciresinol,
relative to the total weight of the extract.

2. The sublingual composition according to claim 1, comprising from about 10% to about 50 % by weight, of said extract, relative to the total weight of the composition.

3. The sublingual composition according to claim 1 or 2, further comprising thickeners, sweeteners, disintegrating agents, dispersing agents, lubricants, and/or flow agents.

4. The sublingual composition according to any one of claims 1 to 3, comprising:
- from about 10 % to about 50 %, preferably from about 10 % to about 40 %, more preferably from about 10 % to about 20 %, by weight of said extract;
- from about 40 % to about 90 %, preferably from about 50 % to about 80 %, by weight of microcrystalline cellulose;
- from about 0.01 % to about 2 %, preferably from about 0.50 % to about 1.50 %, by weight of reticulated sodium carboxymethyl-cellulose; and
- from about 0.01 % to about 2 %, preferably from about 0.50 % to about 1.50 %, by weight of magnesium stearate;
relative to the total weight of the composition.

5. The sublingual composition according to claim any one of claims 1 to 4 further comprising at least one active ingredient for respiratory wellness and/or at least one dietary supplement.

6. The sublingual composition according to claim 5, wherein the at least one active ingredient for respiratory wellness is chosen among an extract from *Abies, Abies siribica, Acerola, Achellea millefolium, Allium ursinum, Althaea officinalis, Andrographis paniculata,* anise, apple, *Artemisia cina, Arum korolkowii, Asperula humifusa, Astragalus, Avenca, Aalsam peru, Aiebersteinia multifidi,* bishop's weed, black spruce, black seed, blueberry, borage, *Brazilian peppertree,* butterbur, *Camellia sinensis,* carapia, catnip, caraway, *Cinnamomum verum, Cinnamomum zeylanicum,* citrus, *Citrus paradisi, Citrus sinenseschamomile,* chaparral, chlorella, *Coleus forskohlii,* coltsfoot, copaiba, *Curcuma longa, Cymbopognon citratus, Cychopelarrium intybus,* dandelion, danshen, *Drosera, echinacea,* embauba, *English ivy,* eucalyptus, *Cucapyptus radiate, Espinheira, Evening primrose, Filipendula ulmaria,* fir, forsythia, *Fucus vesiculosus,* garlic, gelsemium, *Genipa americana, Gotu kola,* ginkgo, ginseng, great plantain, grindelia, *Guaco, mikania glomerata spreng,* guandu, hawthorn, *Hedera helix,* horseradish, horsetail, *Indian frankincense,* juazeiro, *Juniperus seravschanica, Justicia paniculata,* kalanchoe, khella, kiwi, larch, lavender, lavandula, lobelia, *Malpighia glabra, Melaleuca quinquenervia, Malva neglecta, Origanum majorana, Marrubium vulgare,* moringa, myrtus, nettle, *Nigella sativa, Origanum vulgare, Panax ginseng,* pau d'arco, pelargonium, melaleuca, mentha, *Mentha piperita, Origanum majorana, Pelargonium sidoides,* peppermint, *Perilla, picrorhiza,* pine, piper, *Phyllanthus emblica, Plantago major,* plantain, *Plectranthus barbatus, Primula veris,* quillaja, red clover, rosemary, rosmarinus, *Salvia officinalis,* samambaia, schisandra, *Siraitia grosvenorii, Soia isoflavoni, Sophora japonica,* sorrel, soy, *Spirulina platensis,* sweet orange, *Syzygium aromaticum, Taraxacum officinale, Terminalia chebula, Terminalia belerica, Thymus serpyllum, Thymus vulgaris,* tomato, *Tussilágo fárfara,* vassourinha, *Verbascum thapsus, Viburnum lantana,* white horehound, and *Yerba santa,* and/or chosen among alpha-linolenic acid, alpha-lipoic acid, arabinogalactan, arginine, balsam tolu, beta-glucan, bifidobacteria, brewer's yeast, bromelain, caffeine, choline, coenzyme Q-10, cordyceps, eicosapentaenoic acid (EPA), forskolin, fish oil, gamma-aminobutyric acid (GABA), glutathione, green mold, green-lipped mussel, inositol, lactobacillus, linoleic acid, lutein, lycopene, matico, N-acetylcysteine (NAC), pyridoxine, propolis, pycnogenol, quail egg, quercetin, resveratrol, saffron, selenium, serrapeptase, shark liver, umckaloabo, whey protein, yogurt, and zeaxanthin.

7. The sublingual composition according to claim 5, wherein the at least one dietary supplement is ascorbic acid, a vitamin or a mineral chosen among copper, calcium, magnesium, zinc, vitamin A, vitamin B1, vitamin B2, vitamin B3, vitamin B5, vitamin B6, vitamin B8, vitamin B9, vitamin B12, vitamin C, vitamin D, vitamin E, vitamin K, iron, iodine, manganese, and molybdenum.

8. Non-therapeutic use of a sublingual composition as defined in any one of claims 1 to 7, as a food supplement.

9. Non-therapeutic use according to claim 8, for improving the respiratory wellness.

10. Non-therapeutic use according to claim 8, as sport food supplement, especially for improving endurance.

11. A sublingual composition as defined in any one of claims 1 to 7, for use for relieving joint pain and/or muscular pain and cramps.

12. A sublingual composition as defined in any one of claims 1 to 7, for use for improving libido and/or fertility.

13. Kit comprising a sublingual composition as defined in any one of claims 1 to 7 and a package leaflet or user instructions including the information that said composition is to be used for the respiratory wellness, and/or for joint pain and/or muscular pain and cramps and/or endurance, and/or libido and/or fertility.

## Patentansprüche

1. Eine sublinguale Zusammensetzung, umfassend mindestens etwa 10 Gewichtsprozent eines Teils von Zweigextrakten einer Mischung aus *Albies alba* und *Picea abies,* bezogen auf das Gesamtgewicht der Zusammensetzung, wobei der Teil des Zweigextraktes mindestens etwa 50 Gewichtsprozent, vorzugsweise mindestens 70 Gewichtsprozent, mehr bevorzugt 90 Gewichtsprozent Polyphenole und weniger als etwa 10 Gewichtsprozent Terpene, bezogen auf das Gesamtgewicht des Extraktes umfasst, und wobei die Polyphenole
- von etwa 25 bis etwa 50 Gewichtsprozent Hydroxymatairesinol,
- von etwa 5 bis etwa 15 Gewichtsprozent Lariciresinol, und
- von etwa 5 bis etwa 15 Gewichtsprozent Secoisolariciresinol,
bezogen auf das Gesamtgewicht des Extraktes umfassen.

2. Die sublinguale Zusammensetzung nach Anspruch 1, umfassend etwa 10 bis 50 Gewichtsprozent des Extraktes, bezogen auf das Gesamtgewicht der Zusammensetzung.

3. Die sublinguale Zusammensetzung nach Anspruch 1 oder 2, ferner umfassend Verdickungsmittel, Süßungsmittel, Aufschlussmittel, Dispersionsmittel, Schmiermittel und/oder Flussmittel.

4. Die sublinguale Zusammensetzung nach einem der Ansprüche 1 bis 3, umfassend:
- von etwa 10 bis etwa 50 Gewichtsprozent, vorzugsweise von etwa 10 bis etwa 40 Gewichtsprozent, mehr bevorzugt von etwa 10 bis etwa 20 Gewichtsprozent des Extraktes;
- von etwa 40 bis etwa 90 Gewichtsprozent, vorzugsweise von etwa 50 bis etwa 80 Gewichtsprozent mikrokristalliner Zellulose;
- von etwa 0,01 bis etwa 2 Gewichtsprozent, vorzugsweise von etwa 0,50 bis etwa 1,50 Gewichtsprozent retikulierter Natriumcarboxymethylzellulose; und
- von etwa 0,01 bis etwa 2 Gewichtsprozent, vorzugsweise von etwa 0,50 bis etwa 1,50 Gewichtsprozent Magnesiumstearat;
bezogen auf das Gesamtgewicht der Zusammensetzung.

5. Die sublinguale Zusammensetzung nach einem der Ansprüche 1 bis 4, ferner umfassend mindestens einen aktiven Inhaltsstoff für das Wohlbefinden der Atemwege und/oder mindestens ein Nahrungsergänzungsmittel.

6. Die sublinguale Zusammensetzung nach Anspruch 5, wobei der mindestens eine aktive Inhaltsstoff für das Wohlbefinden der Atemwege ausgewählt ist aus einem Extrakt von *Abies, Abies siribica, Acerola, Achella millefolium, Allium ursinum, Althaea officinalis, Andrgraphics paniculata,* Anis, Apfel, *Artemisia cina, Arum korolkowii, Asperula humifusa, Astragalus, Avenca, Aalsam peru, Aiebersteinia multifidi,* Bischofskraut, Schwarzfichte, Schwarzkümmel, Blaubeere, Borretsch, *Brazilian peppertree,* Pestwurz, *Camellia sinensis,* Carapia, Katzenminze, Kümmel, *Cinnamomum verum, Cinnamomum zeylanicum,* Zitruspflanze, *Citrus paradisi, Citrus sinenseschamomile,* Kapern, Chlorella, *Coleus forskohlii,* Huflattich, Copaiba, *Curcuma longa, Cymbopognon citratus, Cychopelarrium intybus,* Löwenzahn, Rotwurzel Salbei, *Drosera, Echinacea,* Ameisenbaum, *English ivy,* Eukalyptus, *Cucapyptus radiate, Espinheira, Evening primrose, Filipendula ulmaria,* Tanne, Forsythie, *Fucus vesiculosus,* Knoblauch, Jasmin, *Genipa americana, Gotu kola,* Ginkgo, Ginseng, Breitwegerich, Grindelia, *Guaco, Mikania glomerata spreng,* Guandu, Weißdorn, *Hedera helix,* Meerrettich, Schachtelhalm, *Indian frankincense,* Juazeiro, *Juniperus seravschanica, Justicia paniculata,* Kalanchoe, Khella, Kiwi, Lärche, Lavendel, echter Lavendel, Lobelien, *Malpigghia glabra, Melaleuca quinquenervia, Malva neglecta, Origanum vulgare, Panax ginseng,* Pau d'Arco, Pelargonien, Myrtenheide, Minze, *Mentha piperita, Origanum majorana, Pelargonium sidoides,* Pfefferminze, *Perilla, Picrorhiza,* Kiefer, Pfeffer, *Phyllanthus emblica, Plantago major,* Wegerich, *Plectranthus barbatus, Primula veris,* Quillaja, Rotklee, Rosmarin, *Salvia officinale,* Samambaia, Schisandra, *Siraitia grosvenorii, Soia isoflavoni, Sophora japonica,* Sauerampfer, Soja, *Spirulina platensis,* süße Orange, *Syzygium aromaticum, Taraxacum officinale, Terminalia chebula, Terminalia belerica, Thymus serpyllum, Thymus vulgaris,* Tomate, *Tussilágo fárfara,* Vassourinha, *Verbascum thapsus, Viburnum lantana,* weißer Andorn und *Yerba santa,* und/oder ausgewählt aus Alpha-Linoleninsäure, Alpha-Liponsäure, Arabinogalaktan, Arginin, Balsam-Tolu, Beta-Glucan, Bifidobakterium, Brauhefe, Bromelain, Koffein, Cholin, Coenzym Q10, Cordyceps, Eikosapentaensäure (EPA), Forskolin, Fischöl, Gamma-Aminobuttersäure (GABA), Glutathion, grüner Schimmel, grünlippige Muschel,Inositol, Lactobacillus, Linolsäure, Lutein, Lycopin, Matico, N-Acetylcystein (NAC), Pyridoxin, Propolis, Pyknogenol, Wachtelei, Quercetin, Resveratol, Safran, Selenium, Serrapeptase, Haifischleber, Umckaloabo, Molkeeiweiß, Yogurt und Zeaxanthin.

7. Die sublinguale Zusammensetzung nach Anspruch 5, wobei das mindestens eine Nahrungsergänzungsmittel Ascorbinsäure, ein Vitamin oder ein Mineral ausgewählt aus Kupfer, Kalzium, Magnesium, Zink, Vitamin A, Vitamin B1, Vitamin B2; Vitamin B3, Vitamin B5, Vitamin B6, Vitamin B9, Vitamin B12, Vitamin C, Vitamin D, Vitamin E, Vitamin K, Eisen, Jod, Mangan und Molybden ist.

8. Nicht-therapeutische Verwendung einer sublingualen Zusammensetzung, wie in einem der Ansprüche 1 bis 7 definiert, als Lebensmittelzusatzstoff.

9. Nicht-therapeutische Verwendung nach Anspruch 8 zur Verbesserung des Wohlbefindens der Atemwege.

10. Nicht-therapeutische Verwendung nach Anspruch 8 als Sport-Lebensmittelzusatzstoff, insbesondere zur Verbesserung der Ausdauer.

11. Eine sublinguale Zusammensetzung, wie in einem der Ansprüche 1 bis 7 definiert, zur Verwendung zur Linderung von Gelenkschmerzen und/oder Muskelschmerzen und Krämpfen.

12. Eine sublinguale Zusammensetzung, wie in einem der Ansprüche 1 bis 7 definiert, zur Verwendung zur Verbesserung der Libido und/oder Fruchtbarkeit.

13. Kit, umfassend eine sublinguale Zusammensetzung, wie in einem der Ansprüche 1 bis 7 definiert, und eine Packungsbeilage oder Gebrauchsanweisung einschließlich der Information, dass die Zusammensetzung zum Wohlbefinden der Atemwege und/oder bei Gelenkschmerzen und/oder bei Muskelschmerzen und Krämpfen und/oder zur Ausdauer und/oder Libido und/oder Fruchtbarkeit verwendet werden soll.

## Revendications

1. Composition sublinguale comprenant au moins environ 10 % en poids d'une partie d'extrait de branches provenant d'un mélange d'*Abies alba* et de *Picea abies,* par rapport au poids total de la composition, dans laquelle ladite partie d'extrait de branches comprend au moins environ 50 %, de préférence au moins 70 %, mieux encore au moins 90 % en poids de polyphénols et moins d'environ 10 % en poids de terpènes, par rapport au poids total de l'extrait, et dans laquelle les polyphénols comprennent :
- d'environ 25 % à environ 50 % en poids d'hydroxymatairésinol,
- d'environ 5 % à environ 15 % en poids de laricirésinol, et
- d'environ 5 % à environ 15 % en poids de sécoisolaricirésinol,
par rapport au poids total de l'extrait.

2. Composition sublinguale selon la revendication 1, comprenant d'environ 10 % à environ 50 % en poids dudit extrait, par rapport au poids total de la composition.

3. Composition sublinguale selon la revendication 1 ou 2, comprenant en outre des épaississants, édulcorants, délitants, dispersants, lubrifiants, et/ou agents d'écoulement.

4. Composition sublinguale selon l'une quelconque des revendications 1 à 3, comprenant :
- d'environ 10 % à environ 50 %, de préférence d'environ 10 % à environ 40 %, mieux encore d'environ 10 % à environ 20 % en poids dudit extrait ;
- d'environ 40 % à environ 90 %, de préférence d'environ 50 % à environ 80 % en poids de cellulose microcristalline ;
- d'environ 0,01 % à environ 2 %, de préférence d'environ 0,50 % à environ 1,50 % en poids de carboxyméthylcellulose sodique réticulée ; et
- d'environ 0,01 % à environ 2 %, de préférence d'environ 0,50 % à environ 1,50 % en poids de stéarate de magnésium ;
par rapport au poids total de la composition.

5. Composition sublinguale selon l'une quelconque des revendications 1 à 4, comprenant en outre au moins un principe actif pour le bien-être respiratoire et/ou au moins un complément alimentaire.

6. Composition sublinguale selon la revendication 5, dans laquelle l'au moins un principe actif pour le bien-être respiratoire est choisi parmi les extraits d'*Abies, Abies siribica, Acerola, Achellea millefolium, Allium ursinum, Althaea officinalis, Andrographis paniculata,* l'anis, la pomme, *Artemisia cina, Arum korolkowii, Asperula humifusa, Astragalus, Avenca, Aalsam peru, Aiebersteinia multifidi,* la petite angélique, l'épinette noire, la nigelle, la myrtille, la bourrache, le poivrier du Brésil, le pétasite, *Camellia sinensis,* le carapia, l'herbe à chat, le carvi, *Cinnamomum verum, Cinnamomum zeylanicum,* les agrumes, *Citrus paradisi, Citrus sinenseschamomile,* le chaparral, la chlorelle, *Coleus forskohlii,* le tussilage, le copaïer, *Curcuma longa, Cymbopognon citratus, Cychopelarrium intybus,* le pissenlit, le danshen, *Drosera, echinacea,* le bois trompette, le lierre commun, l'eucalyptus, *Cucapyptus radiate, Espinheira,* l'onagre, *Filipendula ulmaria,* le sapin, le forsythia, *Fucus vesiculosus,* l'ail, le gelsemium, *Genipa americana, Gotu kola,* le ginkgo, le ginseng, le grand plantain, le grindelia, *Guaco, mikania glomerata spreng,* le guandu, l'aubépine, *Hedera helix,* le raifort, la prêle, *Indian frankincense,* le juazeiro, *Juniperus seravschanica, Justicia paniculata,* le kalanchoe, le khella, le kiwi, le mélèze, la lavande, le lavandula, la lobélie, *Malpighia glabra, Melaleuca quinquenervia, Malva neglecta, Origanum majorana, Marrubium vulgare,* le moringa, la myrte, l'ortie, *Nigella sativa, Origanum vulgare, Panax ginseng,* le lapacho, le pélargonium, le nialoui, la menthe, *Mentha piperita, Origanum majorana, Pelargonium sidoides,* la menthe poivrée, *Perilla, picrorhiza,* le pin, le piment, *Phyllanthus emblica, Plantago major,* le plantain, *Plectranthus barbatus, Primula veris,* le bois de panama, le trèfle rouge, le romarin, *Rosmarinus, Salvia officinalis,* le samambaia, la schisandre, *Siraitia grosvenorii, Soia isoflavoni, Sophora japonica,* l'oseille, le soja, *Spirulina platensis,* l'orange douce, *Syzygium aromaticum, Taraxacum officinale, Terminalia chebula, Terminalia belerica, Thymus serpyllum, Thymus vulgaris,* la tomate, *Tussilago fárfara,* le scopaire doux, *Verbascum thapsus, Viburnum lantana,* le marrube blanc, et *Yerba santa,* et/ou choisi parmi l'acide alpha-linolénique, l'acide alpha-lipoïque, l'arabinogalactane, l'arginine, le baume de Tolu, le bêta-glucane, les bifidobactéries, la levure de bière, la bromélaïne, la caféine, la choline, la coenzyme Q-10, le cordyceps, l'acide éicosapenténoïque (EPA), la forskoline, l'huile de poisson, l'acide gamma-aminobutyrique (GABA), le glutathion, la moisissure verte, la moule verte, l'inositol, les lactobacilles, l'acide linoléique, la lutéine, le lycopène, le matico, la N-acétylcystéine (NAC), la pyridoxine, la propolis, le pycnogénol, les œufs de caille, la quercétine, le resvératrol, le safran, le sélénium, la serrapeptase, le foie de requin, l'umckaloabo, les protéines lactosériques, le yaourt, et la zéaxanthine.

7. Composition sublinguale selon la revendication 5, dans laquelle l'au moins un complément alimentaire est l'acide ascorbique, une vitamine ou un minéral choisi parmi le cuivre, le calcium, le magnésium, le zinc, la vitamine A, la vitamine B1, la vitamine B2, la vitamine B3, la vitamine B5, la vitamine B6, la vitamine B8, la vitamine B9, la vitamine B12, la vitamine C, la vitamine D, la vitamine E, la vitamine K, le fer, l'iode, le manganèse, et le molybdène.

8. Utilisation non thérapeutique d'une composition sublinguale telle que définie dans l'une quelconque des revendications 1 à 7, en tant que complément alimentaire.

9. Utilisation non thérapeutique selon la revendication 8, pour améliorer le bien-être respiratoire.

10. Utilisation non thérapeutique selon la revendication 8, en tant que complément alimentaire pour le sport, en particulier pour améliorer l'endurance.

11. Composition sublinguale selon l'une quelconque des revendications 1 à 7, pour une utilisation dans le but de soulager une douleur articulaire et/ou une douleur musculaire et des crampes.

12. Composition sublinguale selon l'une quelconque des revendications 1 à 7, pour une utilisation dans le but d'améliorer la libido et/ou la fertilité.

13. Trousse comprenant une composition sublinguale telle que définie dans l'une quelconque des revendications 1 à 7 et une notice ou un mode d'emploi incluant l'information selon laquelle ladite composition est destinée à être utilisée pour le bien-être respiratoire, et/ou pour une douleur articulaire et/ou une douleur musculaire et des crampes et/ou l'endurance, et/ou la libido et/ou la fertilité.
